# EUROPEAN PATENT APPLICATION

(11) **EP 1 859 775 A1**
(43) Date of publication of application: **28.11.2007**
(21) Application number: 06728749.0
(22) Date of filing: 08.03.2006
(51) Int. Cl.: A61K 8/26, A61K 8/64, A61K 8/92, A61Q 15/00

(54) **OIL-BASED EXTERNAL PREPARATION FOR SKIN**

(30) Priority: 10.03.2005 JP 2005067074
(71) Applicant: Shiseido Company, Limited, Chuo-ku Tokyo 104-8010 (JP)
(72) Inventor: ARAKI, Hidefumi, c/o SHISEIDO RESEARCH CENTER, Yokohama-shi, Kanagawawa, 2248558 (JP); ISHINO, Hirokazu, c/o SHISEIDO RESEARCH CENTER, Yokohama-shi, Kanagawa, 2248558 (JP)
(74) Representative: Merkle, Gebhard
(86) International application number: PCT/JP2006/304430
(87) International publication number: WO 2006/095753

(57) **Abstract**

The invention provides an oil-based skin treatment composition that contains antibacterial zeolite, an oil component, and glutathione and/or L-cysteine.

The object of the invention is to provide an oil-based skin treatment composition that is blended with antibacterial zeolite and that has excellent washability when it has adhered to articles of clothing.

## Description

### TECHNICAL FIELD

The present invention relates to an oil-based skin treatment composition. More specifically, it relates to oil-based skin treatment compositions that contain antibacterial zeolite and that have improved washability after adhering to clothing.

### BACKGROUND ART

Antibacterial zeolite has been blended with skin treatment compositions such as cosmetics and quasi drugs as a preservative and a deodorant.

For example, an antibacterial spray composition (Patent Document 1) and deodorant cosmetic (Patent Document 2) that contain antibacterial zeolite have been developed. A technology in which silicone is blended with antibacterial zeolite has been disclosed as a deodorant cosmetic with improved ability to resist discoloration (Patent Document 3). There also has been disclosed a skin treatment composition that contains antibacterial zeolite and polyoxyethylene polyoxypropylene 2-decyltetradecyl ether in order to increase the washability of the stain.

[Patent Document 1] JP S63-250325 A
[Patent Document 2] JP H8-26956 A
[Patent Document 3] JP H8-92051 A
[Patent Document 4] W0 2004/037220

### DISCLOSURE 0F INVENTION

### [Problem that the present invention aims to solve]

Antibacterial zeolite by itself is a stable skin treatment composition component. However, the inventors of the present application and others have found that when an oil-based endemic liniment that contains antibacterial zeolite gets onto clothing, it is difficult to remove the oil stain even by washing.

The inventors of the present application also recognized that when oil-based antiperspirant cosmetics that contain antibacterial zeolite get on clothing, and sweat and direct sunlight, and so on, cause unfavorable discoloration that results in oil stains that are difficult to remove even by washing.

Further, particularly in oil-based antiperspirant cosmetics containing aluminum hydroxychloride, a color change that is unfavorable for the appearance of the cosmetic occurs when antibacterial zeolite is included as a preservative or a deodorant, and the inventors of the present application confirmed that the discolored oil stains that occur when this comes into contact with clothing are particularly difficult to remove.

The inventors conducted earnest research in light of the foregoing problem, and surprisingly found that oil-based skin treatment compositions containing antibacterial zeolite, glutathione and/or L-cysteine, and an oil component are easily washed away from clothing to which they have adhered and caused an oil stain that is difficult to remove, the invention has been completed based on these discoveries.

It is an object of the invention to provide an oil-based skin treatment composition containing antibacterial zeolite that has improved washability when it has adhered to clothing.

### [Means to solve the Problem]

That is, the invention provides an oil-based skin treatment composition that contains antibacterial zeolite, glutathione and/or L-cysteine, and an oil component.

The invention also provides an oil-based skin treatment composition that has the characteristics of the above oil-based skin treatment composition and is characterized in that glutathione and/or L-cysteine are each contained to 0.01 to 5.0 wt% of the total weight of the oil-based skin treatment composition.

The invention also provides an oil-based skin treatment composition that has the characteristics of the above oil-based skin treatment composition and is characterized in that it further includes an aluminum compound in powder form.

### [Effects of the invention]

The oil-based skin treatment composition of the invention exhibits a noticeable improvement in washability when it has adhered to and soiled to clothing. Also, because it is oil-based formula, oil soluble drugs etc. can be stably blended therewith.

### BRIEF DESCRIPTION OF DRAWINGS

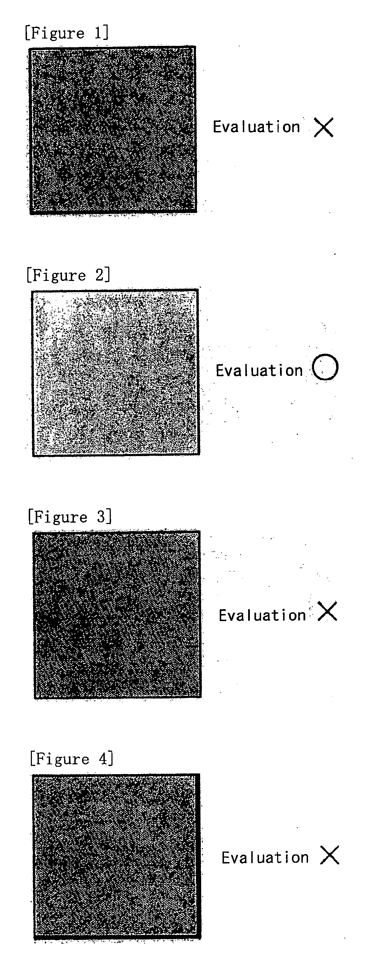

[Figure 1] A photograph of "Soiling Clothes After Exposure to Sunlight" of Example 1.
[Figure 2] A photograph of the "Soiling Clothes After Washing" of Example 1.
[Figure 3] A photograph of "Soiling Clothes After Exposure to Sunlight" of Comparative Example 14.
[Figure 4] A photograph of the "Soiling Clothes After Washing" of Comparative Example 14.

### BEST MODE FOR CARRYING OUT THE INVENTION

The invention is described in detail below.

The antibacterial zeolite used in this invention is zeolite that holds antibacterial metal ions in an ion-exchangeable area of the zeolite.
That is, some or all of the exchangeable ions of the zeolite have been substituted by antibacterial metal. In this invention, it is preferable that the zeolite has been substituted by antibacterial metal ions as well as ammonium ion.

The zeolite may be natural zeolite or synthetic zeolite. Zeolite is generally an aluminosilicate that has a three dimensional skeletal structure, and is represented by the general formula XM_{2/n}0·Al₂O₃·YSiO₂·ZH₂O. In this general formula, M represents the ion-exchangeable ion, and normally this is a monovalent or divalent metal ion. n represents the valency of the (metal) ion. X and Y represent coefficients of the metal oxide and the silica, respectively, and Z represents the number of water of crystallization.

Specific examples of zeolite include A-type zeolite, X-type zeolite, Y-type zeolite, T-type zeolite, high silica zeolite, sodalite, mordenite, analcime, crinoptyrolite, chabasite, and erionite. The ion exchange capacities of these zeolites are: 7 meq/g for A-type zeolite, 6.4 meq/g for X-type zeolite, 5 meq/g for Y-type zeolite, 3.4 meq/g for T-type zeolite, 11.5 meq/g for sodalite, 2.6 meq/g for mordenite, 5 meq/g for analcime, 2.6 meq/g for crinoptyrolite, 5 meq/g for chabasite, and 3.8 meq/g for erionite. Any of these has capacity sufficient for ion exchange with antibacterial metal ions and/or ammonium ions.

Examples of exchangeable ions in zeolite include sodium ions, calcium ions, potassium ions, magnesium ions, and iron ions.
Examples of the antibacterial metal ions that may replace these ions include silver, copper, zinc, mercury, tin, lead, bismuth, cadmium, chromium, and thallium ions, preferably silver, copper, or zinc ions, and more preferably silver ions.

For the sake of antibacterial activity, the content of the antibacterial ions is preferably 0.1-15 wt% of the zeolite.

For example, antibacterial zeolite containing 0.1-15 wt% of silver ion and 0.1-8 wt% of copper ion or zinc ion is preferable. On the other hand, zeolite can contain up to 20 wt% of ammonium ion; however, in order to effectively prevent discoloration of the zeolite, 0.5-5 wt% of ammonium ion is preferable and 0.5-2 wt% is more preferable. It should be noted that "wt%" means the weight percent in 110°C dry standard zeolite.

In the present invention, commercial products can be used for the antibacterial zeolite, and it is also possible to prepare the antibacterial zeolite by the following method, for example.
That is, zeolite is brought into contact with a mixed solution containing antibacterial metal ions such as silver ions, copper ions, or zinc ions, that has been prepared in advance, to replace the aforementioned ions with the exchangeable ions in the zeolite.

The contacting can be achieved by a batch method or continuous method (column method, for example) for 3-24 hours, preferably 10-24 hours, at 10-70°C, preferably 40-60°C.

The aforementioned mixed solution should be adjusted to a pH of 3-10 and more preferably 5-7. This adjustment is preferable because it can prevent the precipitation of silver oxides, etc., on the zeolite surface or in its fine pores. The ions in the mixed aqueous solution are usually supplied as salts. For example, the silver ions can be from silver nitrate, silver sulfate, silver perchlorate, silver acetate, diamminesilver nitrate, or diamminesilver sulfate, etc.; the copper ions can be from copper(II) nitrate, copper perchlorate, copper acetate, potassium tetracyanocuprate, or copper sulfate, etc.; the zinc ions can be from zinc(II) nitrate, zinc sulfate, zinc perchlorate, zinc thiocyanate, or zinc acetate, etc.; the mercury ions can be from mercury perchlorate, mercury nitrate, or mercury acetate; the tin ions can be from tin sulfate etc.; the lead ions can be from lead sulfate or lead nitrate, etc.; the bismuth ions can be from bismuth chloride or bismuth iodide, etc.; the cadmium ions can be from cadmium perchlorate, cadmium sulfate, cadmium nitrate, or cadmium acetate; the chromium ions can be from chromium perchlorate, chromium sulfate, chromium ammonium sulfate, chromium nitrate, etc.; and the thallium ions can be from thallium perchlorate, thallium sulfate, thallium nitrate, or thallium acetate, etc.

The antibacterial metal ion content of the zeolite can be suitably controlled by adjusting the concentration of each ion (salt) in said mixed aqueous solution.

For example, if the antibacterial zeolite is to contain silver ions, then an antibacterial zeolite whose silver ion content is 0.1-5% can be obtained by setting the silver ion concentration in said mixed aqueous solution to 0.002 mole/l to 0.15 mole/l.
Moreover, if the antibacterial zeolite also contains copper and zinc ions, then an antibacterial zeolite having 0.1-8 wt% copper ion content and 0.1-8 wt% zinc ion content can be suitably obtained by setting the copper ion concentration of said mixed aqueous solution to 0.1 mole/l to 0.85 mole/l and its zinc ion concentration to 0.15 mole/l to 1.2 mole/l.

In addition to the above mixed solution, it is also possible to use individual aqueous solutions each containing one of the ions and to successively contact the antibacterial zeolite with these solutions to exchange ions therewith. The concentration of the ions in the aqueous solutions can be determined in accordance with the concentration of those ions in said mixed aqueous solution.

After the completion of the ion exchange, the zeolite is thoroughly rinsed and then dried. The zeolite is preferably dried at a temperature 105-115°C, or at 70-90°C under a reduced pressure (1-30 Torr).

Ion exchange with organic ions and/or ions such as tin and bismuth for which there an adequate water soluble salt does not exist can be carried out using an organic solvent solution such as an alcohol or acetone to keep slightly soluble basic salts from precipitating.

There are no particular limitations regarding the amount of antibacterial zeolite that is mixed in with the oil-based skin treatment composition. This amount can be suitably determined in accordance with the purpose for this blending and the form that the oil-based skin treatment composition product will take.

For example, when zeolite is included as a preservative, the zeolite is usually added to 0.05-10 wt% of the total weight of the oil-based skin treatment composition.

Alternatively, if zeolite is added as a bactericide, then it is usually added to 0.1-90 wt% of the total amount of the oil-based skin treatment composition, depending on the form that the product will take.
For example, for lotion or cream type oil-based skin treatment compositions, 0.1-20 wt% of the total weight of the oil-based skin treatment composition is preferable; for powder type oil-based skin treatment compositions, 0.5-80 wt% of the total amount of the oil-based skin treatment composition is preferable; for stick type oil-based skin treatment compositions, 0.5-60 wt% of the total weight of the oil-based skin treatment composition is preferable; and for spray type oil-based skin treatment compositions, 0.5-50 wt% is preferable.

Glutathione and L-cysteine are used in the present invention, and these are well-known as skin treatment composition components. However, it was never found that these have the effect of easing the removal of soiling that has occurred in clothing, and these have not heretofore been included as "agents for improving washability" in oil-based skin treatment compositions that contain antibacterial zeolite.
The amount of glutathione and/or L-cysteine to be included is about 0.01 to 5.0 wt% each per total weight of oil-based skin treatment composition. Preferably this is 0.1 to 1.0 wt%. It is also preferable that the two are used together.

The oil-based skin treatment composition of the invention substantially excludes water, and as a base material includes an oil component. There are no particular limitations regarding the oil component as long as it is an oil component that is commonly used in skin treatment compositions. Examples of oil components that can be used are listed below, but preferably silicone oil is used. A volatile silicone oil such as octamethyl cyclotetrasiloxane, decamethyl cyclopentasiloxane, or dodecamethyl cyclohexasiloxane is preferable particularly if the oil-based skin treatment composition is to be used as an antiperspirant power spray. Further, oil-based skin treatment compositions that include a volatile silicone oil as a main component are unlikely to remaining stain after washing.
There are no limitations regarding the amount of oil component that is present. Normally it is about 15 to 60 wt% of the total weight of the oil-based skin treatment composition.

### <Examples of oil components that can be used as the base material of the oil-based skin treatment composition>

Examples of liquid fats and oils include avocado oil, tsubaki oil, turtle fatty acid, macademia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japanese gimlet oil, jojoba oil, germ oil, and triglycerin.

Examples of solid fats and oils include cacao butter, coconut oil, hydrogenated coconut oil, palm oil, palm kernel oil, Japanese core wax nucleus oil, hydrogenated oil, Japanese core wax, and hydrogenated castor oil.

Examples of waxes include beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, P0E cholesterol ether, lanolin fatty acid polyethylene glycol, P0E hydrogenated lanolin ethyl alcohol ether, ceresin, and microcrystalline wax.

Examples of hydrocarbon oils include liquid paraffin, ozocerite, squalane, pristane, paraffin, squalene, and petrolatum.

Examples of higher fatty acids include lauric acid, myristic acid, palmitic acid, stearic acid, behenic acid, oleic acid, undecylenic acid, isostearic acid, linolic acid, linoleic acid, eicosapentaenoic acid (EPA), and docosahexaenoic acid (DHA).

Examples of higher alcohols include straight chain alcohols (for example, lauryl alcohol, cetyl alcohol, stearyl alcohol, behenyl alcohol, myristyl alcohol, oleyl alcohol, and cetostearyl alcohol) and branched chain alcohols (for example, mono stearyl glycerin ether (batyl alcohol), 2-decyltetradecynol, lanolin alcohol, cholesterol, phytosterol, hexyl dodecanol, isostearyl alcohol, and octyldodecanol).

Examples of ester oils include isopropyl myristate, cetyl octanoate, octyldodecyl myristate, isopropyl palmitate, butyl stearate, hexyl laurate, myristil myristate, decyl oleate, hexyldecyl dimethyloctanoate, cetyl lactate, myristil lactate, lanolin acetate, isocetyl stearate, isocetyl isostearate, cholesteryl hydroxy 12-stearate, ethylene glycol di-2-ethylhexanoate, dipentaerythritol fatty acid ester, n-alkylene glycol monoisostearate, neopentyl glycol dicaprate, diisostearyl malate, glyceryl di-2-heptylundecanoate, trimethylolpropane tri-2-ethylhexanoate, trimethylolpropane triisostearate, tetra-2-pentaerythritol ethylhexanoate, glycerin tri-2-ethylhexanoate, glyceryl trioctanoate, glycerin triisopalmitate, trimethylolpropane triisostearate, cetyl 2-ethyl hexanoate, 2-ethylhexyl palmitate, glycerin.trimyristate, tri-2-heptyl undecanoic acid glyceride, methyl castor oil fatty acid, oleyl oleate, aceto glyceride, 2-heptyl undecyl palmitate, diisobutyl adipate, 2-octyldodecyl N-lauroyl-L-glutamate, di-2-heptyl undecyl adipate, ethyl laurate, di-2-ethylhexyl sebacate, 2-hexyl decyl myristate, 2-hexyldecyl palmitate, 2-hexyl decyl adipate, diisopropyl sebacate, 2-ethylhexyl succinate, and triethyl citrate.

Examples of silicone oils include linear polysiloxanes (for example, dimethylpolysiloxane, methylphenyl polysiloxane, and diphenyl polysiloxane), cyclic polysiloxanes (for example, octamethylcyclotetrasiloxane, decamethyl cyclopentasiloxane, and dodecamethyl cyclohexasiloxane), silicone resins forming a three-dimensional network structure, silicone rubbers, and various modified polysiloxanes (amino-modified polysiloxane, polyether-modified polysiloxane, alkyl-modified polysiloxane, and fluorine-modified polysiloxane).

In cases where the oil-based skin treatment composition of the invention is to be employed in applications such as deodorant cosmetics or antiperspirant cosmetics, it is preferable that a powder is included therein. In particular, it is preferable that powders such as starch (rice, corn, potato, wheat), cellulose powder, silicone powder, or powdered aluminum compounds, which act as antiperspirants, are blended therein.
Examples of the powder ingredients include inorganic powders (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, barium sulfate, firing calcium sulfate (calcined gypsum), calcium phosphate, fluorine-apatite, hydroxy apatite, ceramic powder, metallic soaps (for example, zinc myristate, calcium palmitate, and aluminum stearate), and boron nitride); organic powders (for example, polyamide resin powder (nylon powder), polyethylene powder, poly methyl methacrylate powder, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ-iron oxide); inorganic yellow pigments (for example, yellow iron oxide and loess); inorganic black pigments (for example, black iron oxide and low oxides of titanium); inorganic purple pigments (for example, manganese violet, cobalt violet); inorganic green pigments (for example, chromium oxide, chromium hydroxide, and cobalt titanate); inorganic blue pigments (for example, ultramarine blue and Berlin blue); pearl pigment (for example, titania coated mica, titania coated bismuth oxychloride, titania coated talc, coloration titania coated mica, bismuth oxychloride, fish scale flakes); metal powder pigments (for example, aluminum powder, copper powder); organic pigments such as Zr, barium or aluminum rake (for example, organic pigments such as red 201, red 202, red 204, red 205, red 220, red 226, red 228, red 405, orange 203, orange 204, yellow 205, yellow 401 and blue 404, as well as red 3, red 104, red 106, red 227, red 230, red 401, red 505, orange 205, yellow 4, yellow 5, yellow 202, yellow 203, green 3 and blue 1; and natural colors (for example, chlorophyll and β-carotene)).

In addition to the aforementioned essential ingredients, other ingredients that are commonly used in skin treatment compositions, for example one or more of the components illustratively listed below, may suitably blended as necessary in the oil-based skin treatment composition of the present invention, and can be prepared through a conventional method in accordance with the form that the preparation is to take.

Examples of anionic surfactants include fatty acid soaps (for example, sodium laurate and sodium palmitate); higher alkyl sulfuric ester salts (for example, sodium lauryl sulfate and potassium laurylsulfate); alkylether sulfuric ester salts (for example, POE-triethanolamine laurylsulfate and sodium POE-lauryl sulfate); N-acyl sarcosinic acids (for example, sodium N-lauroyl sarcosinate); higher fatty acid ester sulfates (for example, hydrogenated coconut oil aliphatic acid glycerin sodium sulfate); N-acyl glutamates (for example, mono sodium N-lauroylglutamate, disodium N-stearoylglutamate, and sodium N-myristoyl-L-glutamate); sulfated oils (for example, turkey red oil); POE-alkylether carboxylic acid; POE-alkylarylether carboxylate; α-olefin sulfonate; higher fatty acid ester sulfonates; sec-alcohol sulfates; higher fatty acid alkyl amide sulfates; sodium lauroyl monoethanolamine succinates; ditriethanolamine N-palmitoylaspartate; and sodium caseinate.

Examples of cationic surfactants include alkyltrimethylammonium salts (for example, stearyltrimethylammonium chloride and lauryltrimethyl ammonium chloride); alkylpyridinium salts (for example, cetylpyridinium chloride); distearyldimethylammonium chloride dialkyldimethylammonium salt; poly(N,N'-dimethyl-3,5-methylene piperidinium) chloride; alkyl quaternary ammonium salts; alkyl dimethylbenzyl ammonium salts; alkyl isoquinolinium salts; dialkylmorpholine salts; POE alkyl amines; alkyl amine salts; polyamine fatty acid derivatives; amylalcohol fatty acid derivatives; benzalkonium chloride; and benzethonium chloride.

Examples of ampholytic surfactants include: imidazoline type ampholytic surfactants (for example, 2-undecyl-N,N,N-(hydroxyethyl carboxymethyl)-2-imidazoline sodium salt and 2-cocoyl-2-imidazolinium hydroxide-1-carboxyethyloxy 2 sodium salt); and betaine type surfactants (for example, 2-heptadecyl-n-carboxymethyl-n-hydroxyethyl imidazolinium betaine, lauryldimethylaminoacetic acid betaine, alkyl betaine, amide betaine, and sulfobetaine).

Examples of lipophilic nonionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate); glycerin polyglycerin aliphatic acids (for example, mono-cottonseed oil fatty acid glycerin, glyceryl monoerucate, glycerin sesquioleate, glyceryl monostearate, α,α'-glyceryl oleate pyroglutamate, glyceryl mono stearate mono malate); propylene glycol fatty acid esters (for example, propylene glycol monostearate); hydrogenated castor oil derivatives; and glycerin alkylethers.

Examples of hydrophilic nonionic surfactants include: P0E-sorbitan fatty acid esters (for example, POE-sorbitan monooleate, POE-sorbitan monostearate, POE-sorbitan monoolate, and POE-sorbitan tetraoleate); POE sorbitol fatty acid esters (for example, POE sorbitol monolaurate, P0E-sorbitol monooleate, POE-sorbitolpentaoleate, and POE-sorbitol monostearate); POE-glycerin fatty acid esters (for example, P0E-monooleates such as P0E-glycerin monostearate, POE-glycerin monoisostearate, and POE-glycerin triisostearate); POE-fatty acid esters (for example, POE-distearate, P0E-monodioleate, and ethylene glycol distearate); POE-alkylethers (for example, POE-lauryl ether, P0E-oleyl ether, POE-stearyl ether, POE-behenyl ether, POE-2-octyl dodecyl ether, and P0E-cholestanol ether); POE/POP-alkylethers (for example, POE/POP-cetyl ether, POE/POP-2-decyl tetradecyl ether, POE/POP-monobutyl ether, POE/POP-lanolin hydrate, and POE/POP-glycerin ether); POE-castor oil hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamic monoisostearic diester, and POE-hydrogenated castor oil maleic acid); POE-beeswax/lanolin derivatives (for example, POE-sorbitol beeswax); alkanol amides (for example, coconut fatty acid diethanol amide, lauric acid monoethanol amide, and aliphatic acid isopropanol amide); POE-propylene glycol fatty acid esters; POE-alkyl amine; POE-fatty acid amide; sucrose fatty acid ester; alkyl ethoxy dimethylamine oxides; and trioleyl phosphoric acid.

Examples of thickeners include aluminum magnesium silicate, bentonite, hectorite, AlMg silicate (beagum), laponite, and anhydrous silicate.

Examples of ultraviolet absorbents include the following compounds.
(1) Benzoic acid-type ultraviolet absorbents
   For example, paraminobenzoic acid (hereafter abbreviated as PABA), PABA monoglycerin ester, N,N-dipropoxy PABA ethyl ester, N,N-diethoxy PABA ethyl 1 ester, N,N-dimethyl PABA ethyl ester, N,N-dimethyl PABA butyl ester, and N,N-dimethyl PABA ethyl ester.
(2) Anthranilic acid-type ultraviolet absorbents
   For example, homo mentyl-N-acetyl anthranilate.
(3) Salicylic acid-type ultraviolet absorbents
   For example, amyl salicylate, mentyl salicylate, homo mentyl salicylate, octyl salicylate, phenyl salicylate, benzyl salicylate, and p-isopropanol phenyl salicylate.
(4) Cinnamic acid-type ultraviolet absorbents
   For example, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α -cyano-β -phenyl cinnamate, 2-ethylhexyl-α-cyano-β -phenyl cinnamate, and glyceryl mono-2-ethyl hexanoyl-diparamethoxy cinnamate.
(5) Triazine-type ultraviolet absorbents
   For example, bisresorsinyl triazine.
   More specifically, bis{[4-(2-ethylhexyloxy)-2-hydroxy]phenyl}-6-(4-methoxyphenyl)1,3,5-triazine, 2,4,6-tris{4-(20ethylhexyloxycarbonyl) anilino} 1, 3, 5-triazine, etc.
(6) Other ultraviolet absorbents
   For example, 3-(4'-methylbenzylidene)-d,1-camphor,3-benzylidene-d,1-camphor, 2-phenyl-5-methyl benzoxazol, 2-(2'-hydroxy-5'-methylphenyl) benzotriazol, 2-(2'-hydroxy-5'-t-octylphenyl) benzotriazol, 2-(2'-hydroxy-5'-methylphenyl benzotriazol, dibenzaladine, dianisoylmethane, and 4-methoxy-4'-t-butyl dibenzoyl-methane, 5-(3,3-dimethyl-2-norbornylidene)-3-pentane-2-one. Pyridazinone derivatives such as dimorpholino pyridazine.

Examples of sequestering agents include 1-hydroxy ethane-1,1-diphosphonic acid, 1-hydroxy ethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, and edetic acid.

Examples of lower alcohols include ethanol, propanol, isopropanol, isobutyl alcohol, and t-butyl alcohol.

Examples of polysaccharides include cellulose and starch.

Examples of amino acids include neutral amino acids (for example, threonine and cysteine) and basic amino acids (for example, hydroxylysine). Examples of amino acid derivatives include sodium acyl sarcosinate (sodium N-lauroyl sarcosinate), acyl glutamate, sodium acyl β-alanine, glutathione, and pyrrolidone carboxylic acid.

Examples of organic amines include monoethanolamine, diethanolamine, triethanolamine, morpholine, triisopropanolamine, 2-amino-2-methyl-1,3-propanediol, and 2-amino-2-methyl-1-propanol.

Examples of vitamins include vitamins A, B1, B2, B6, C and E as well as their derivatives, pantothenic acid and its derivatives, and biotin.

Examples of the antioxidants include tocopherols, dibutyl hydroxytoluene, butyl hydroxyanisole, and gallic ester.

Examples of antioxidant auxiliary agents include phosphoric acid, citric acid, ascorbic acid, maleic acid, malonic acid, succinic acid, fumaric acid, cephalin, hexameta phosphate, phytic acid, and ethylene diamine tetraacetic acid.

Examples of other possible ingredients include antiseptics (methylparaben, ethylparaben, butylparaben, and phenoxyethanol); antiinflammatory agents (for example, glycyrrhizic acid derivatives, glycyrrhetinic acid derivatives, salicylic acid derivatives, hinokitiol, zinc oxide, and allantoin); whitening agents (for example, creeping saxifrage extract, arbutin, tranexamic acid, L-ascorbic acid, magnesium L-ascorbyl phosphate, L-ascorbic acid glucosie, and potassium 4-methoxysalicylate); various extracts (for example, Phellodendri Cortex, goldthread, lithospermum root, Paeonia lactiflora, Swertia japonica, Birch, sage, loquat, carrot, aloe, Malva sylvestris, Iris, grape, Coix ma-yuen, sponge gourd, lily, saffron, Cnidium officinale, sheng jiang, Hypericum erectum, Ononis, garlic, Guinea pepper, chen pi, Ligusticum acutilobum, and seaweed), activators (royal jelly, photosensitive substances, and cholesterol derivatives); blood circulation promoting agents (for example, nonyl acid valenyl amide, nicotinic acid benzyl esters, nicotinic acid β -butoxy ethyl esters, capsaicin, gingeron, cantharis tincture, Ichthammol, tannic acid, α-borneol, tocopherol nicotinate, inositol hexanicotinate, cyclandelate, cinnarizine, tolazoline, acetylcholine, verapamil, cepharanthine, and γ-orizanol); anti-seborrhea agents (for example, sulfur and thiantol); and antiinflammatory agents (for example, thiotaurine and hypotaurine); and bactericides (for example, benzoic acid and its salts, isopropylmethyl phenol, undecylenic acid and its salts, undecylenic acid monoethanol amide, cetyltrimethyl ammonium chloride, cetylpyridinium chloride, benzalkonium chloride, benzethonium chloride, alkyldiaminoethylglycine chloride, chlorhexidine chloride, orthophenyl phenol, chlorhexidine gluconate, cresol, chloramine T, chlorxylenol, chlorcresol, chlorfenesin, chlorobutanol, 5-chloro-2-methyl-4-isothiazoline-3-one, salicylic acid and its salts, 1,3-dimethylol-5,5-dimethylhidantoin, alkylisoquinolium bromide, domiphen bromide and its salt, sorbic acid and its salts, thymol, thylum, thiram, dehydroacetic acid and its salt, triclosan, trichlorocarbanilide, paraoxybenzoic ester, parachlorphenol, halocarban, pyrogallol, phenol, hexachlorophene, 2-methyl-4-isothiazoline-3-one, NN"-Methylenebis(N'-(3-hydroxymethyl-2,5-dioxo-4-imidazolidinyl)urea), sodium layroylsarcosine, and resorcin).

There are no limitations regarding the form taken by the oil-based skin treatment composition product of the invention; however, it can be used preferably as an antiperspirant cosmetic. It is preferable that an oil-based skin treatment composition of the invention blended with powder is loaded into an aerosol canister along with a commonly known propellant such as LPG or DME, and then used as an antiperspirant powder spray.

### EXAMPLES

The present invention is described in detail below based on examples. The present invention is not limited to these examples. The blend ratio is shown as a wt% unless specified otherwise.

Powder lotion-type antiperspirant lotions were prepared using the ingredients shown in Table 1 and Table 2, and these were each loaded into an aerosol container at an approximately 1:9 ratio of raw liquid (powder lotion) to LPG gas.
This was then uniformly applied (0.06 g/cm²) onto a white colored article of clothing and artificial sweat (water to 100 wt%, sodium chloride 0.8 wt%, acetic acid 0.5 wt%, dibasic sodium phosphate dodecahydrate 0.8 wt%) was added dropwise at 0.012 g/cm², after which it was exposed to sunlight (10 minutes) and the degree of soiling was visually evaluated. This discolored article of clothing was then washed in a washing machine using normal clothes detergent, after which the degree of soiling was visually evaluated. These were determined based on the following standards.

### <Soiling After Exposure to Sunlight (Discoloration Prevention Effect)>

The results of the evaluation of the "soiling after exposure to sunlight (discoloration prevention effect)" are shown in the tables, in which instances where the soiling (discoloration) is lighter than the soiling (discoloration) in Comparative Example 1 and Comparative Example 14 are denoted by an empty circle and instances where this is not the case are denoted by an x.

### <Soiling After Washing (Washability Effect)>

The results of the evaluation of the "soiling after washing (washability effect)" are shown in the tables, in which instances where the soiling (discoloration) is lighter than the above soiling (discoloration) after exposure to sunlight are denoted by an empty circle and instances where this is not the case are denoted by an x.

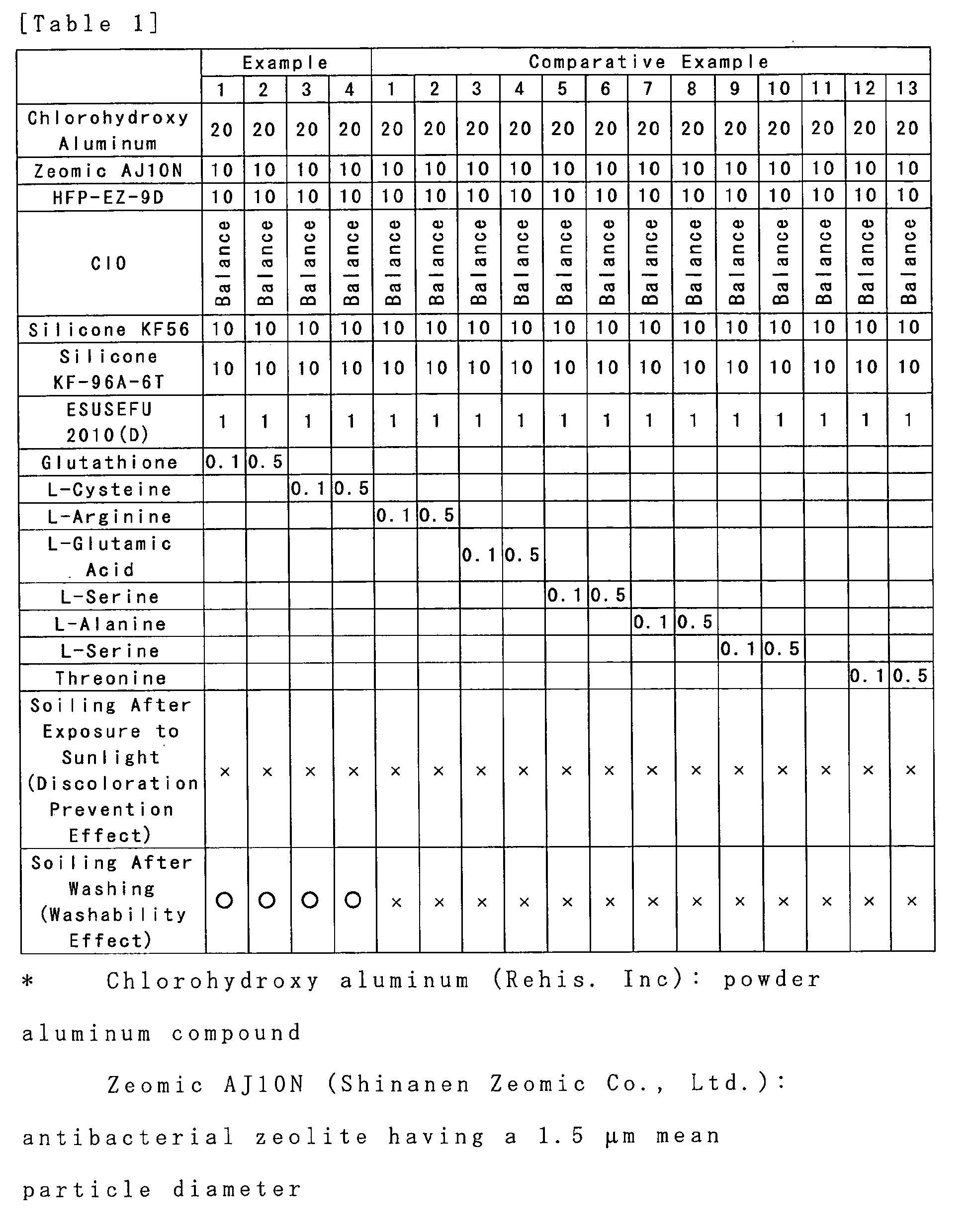

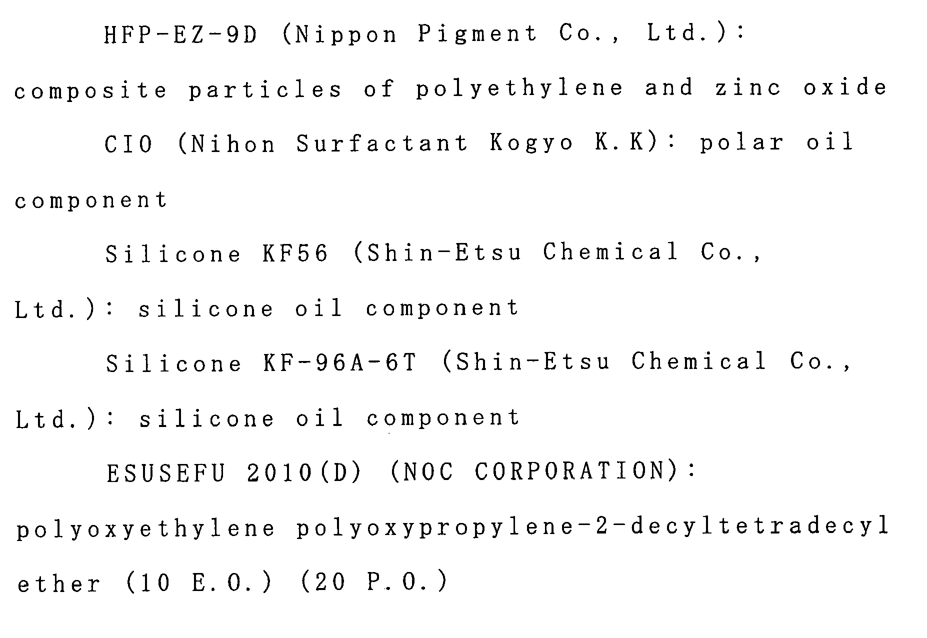

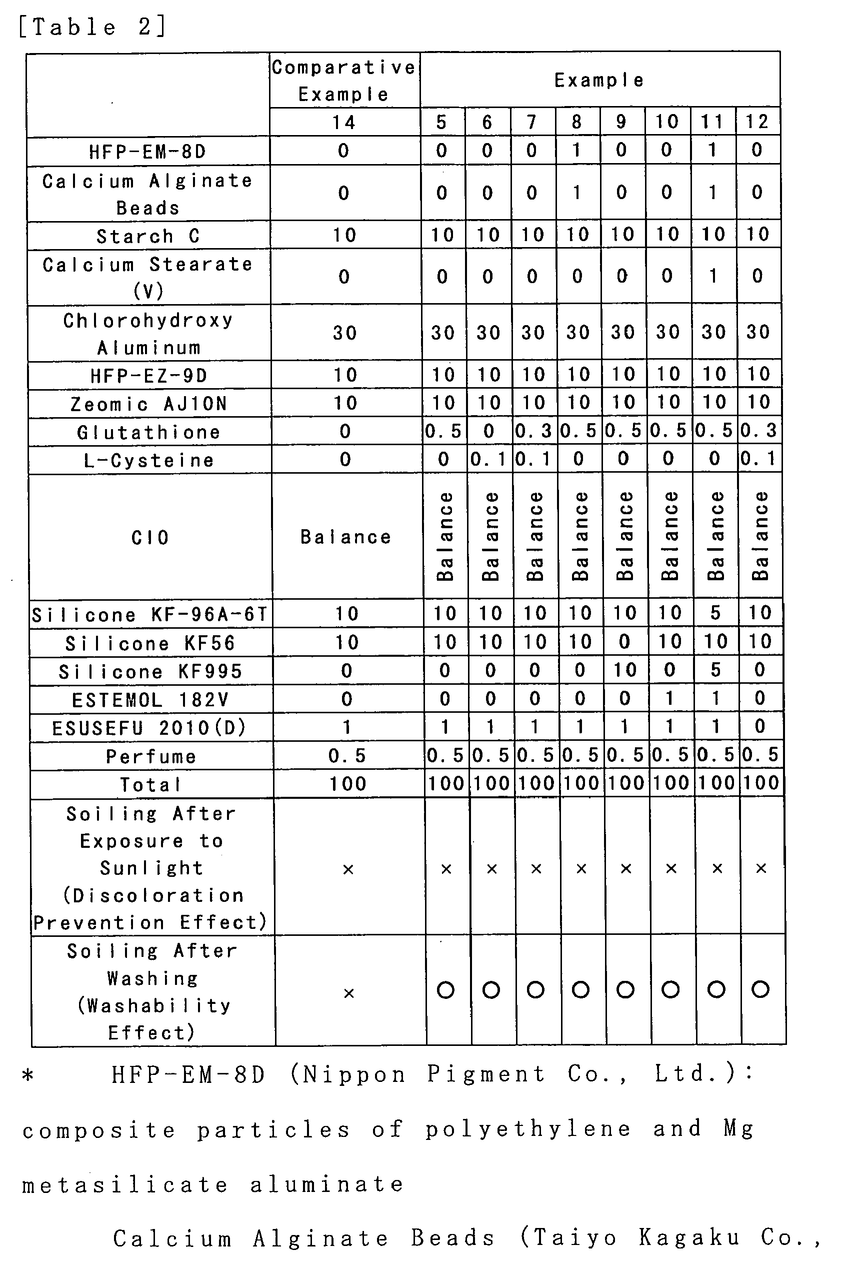

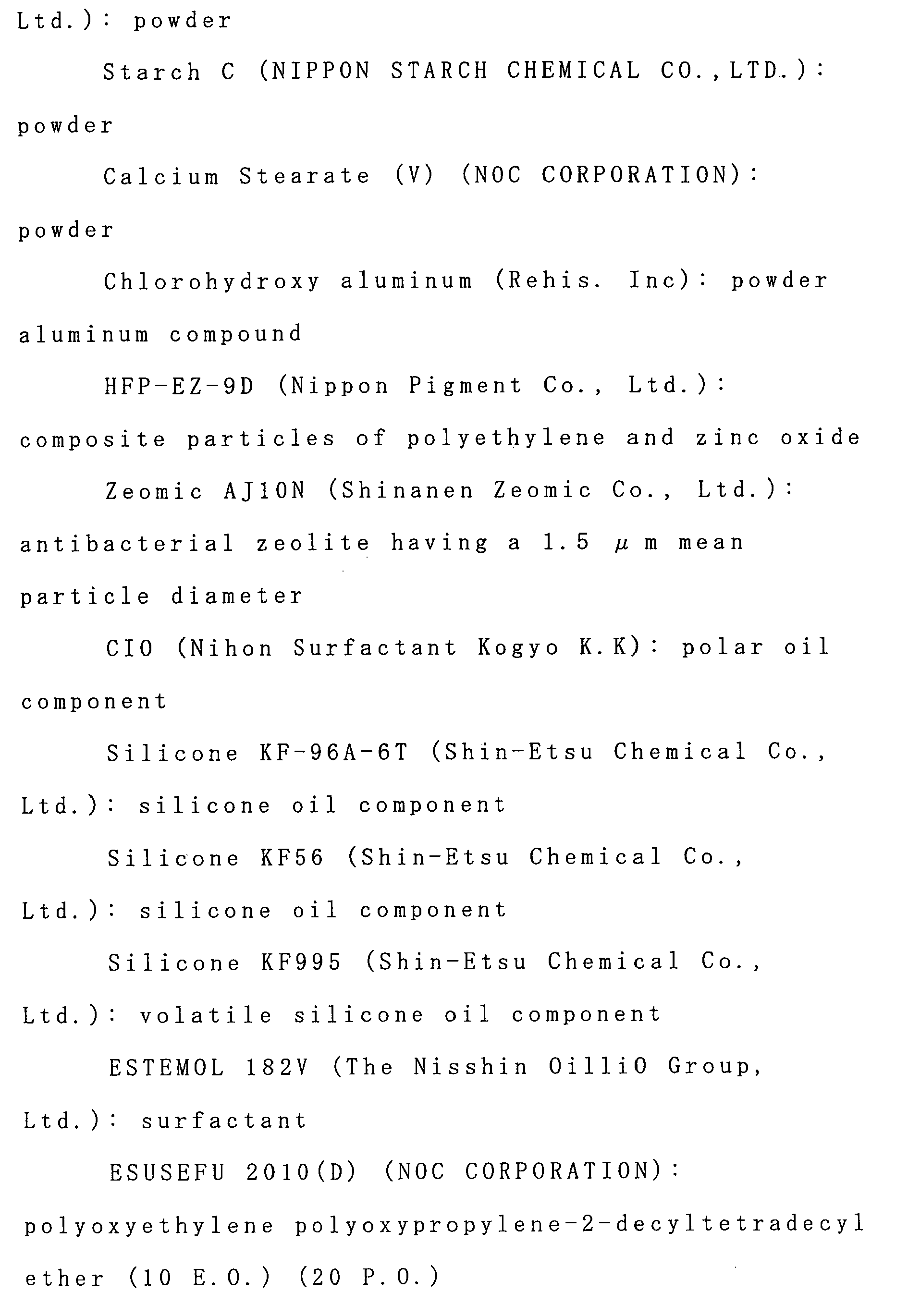

From the results of Table 1 and Table 2, it can be understood that when an oil-based antiperspirant cosmetic blended with antibacterial zeolite has adhered to an article of clothing, then sweat and sunlight, for example, can cause unfavorable soiling (discoloration) that leads to oil stains that are difficult to remove even by washing. In other words, in both the Examples and the comparative examples, the cosmetic causes soiling (discoloration) when it adheres to clothing, and the effect of preventing soiling (discoloration) is not achieved. However, blending glutathione and/or L-cysteine in the oil-based skin treatment composition results in an oil-based skin treatment composition with excellent washability, evidenced by the fact that washing reduces soiling (discoloration) more than when glutathione and/or L-cysteine are not included or when other amino acids have been included.
From the above it is clear that the invention of this application is very useful for oil-based skin treatment compositions that include antibacterial zeolite, which causes soiling (discoloration). Particularly since antiperspirant cosmetics are used on armpits, which are areas where sweating occurs, the cosmetic easily adheres to white colored clothing such as underwear and T-shirts and the soiling to noticeable and for this reason the invention of the present application has particularly large merit.

Other Examples of the invention are illustrated below.

### Example 13 Stick-type Antiperspirant

| | |
|---|---|
| Chlorohydroxy aluminum | 20 wt% |
| Talc | 8 |
| Zinc oxide (zinc white) | 5 |
| Solid petrolatum wax | 2 |
| Stearyl alcohol | 8 |
| Liquid petrolatum | 15 |
| Cyclic dimethyl polysiloxane | 34 |
| Sorbitan fatty acid ester | 1 |
| Zeomic AJ10N | 5 |
| ESUSEFU 2010(D) | 1 |
| L-cysteine | 1 |

A normal method was employed to produce the stick-type antiperspirant. The product had excellent washability from areas where it had adhered to clothing.

### Example 14 Pressed Powder

| | |
|---|---|
| Chlorohydroxy aluminum | 10 wt% |
| Zinc oxide (zinc flower) | 5 |
| Talc | 80.5 |
| Liquid petrolatum | 3 |
| Zeomic AJ10N | 1 |
| Glutathione | 0.5 |

A normal method was used to produce the pressed powder. The product had excellent washability from areas where it had adhered to clothing.

### INDUSTRIAL APPLICABILITY

The present invention is an oil-based skin treatment composition with improved washability when it has adhered to articles of clothing. That is, when the oil-based skin treatment composition has adhered to clothing, then sweat or sunlight may cause discoloration that is difficult to remove, but this oil-based skin treatment composition exhibits the effect that washing the clothing allows the discoloration to be removed with ease and reduces the degree of soiling. The oil-based skin treatment composition of the invention can be particularly favorably used as an antiperspirant cosmetic, which easily stains clothing due to sweat or oil. Also, being oil based, oil-soluble drugs, for example, can be stably blended therein.

## Claims

1. An oil-based skin treatment composition comprising antibacterial zeolite, glutathione and/or L-cysteine, and an oil component.

2. The oil-based skin treatment composition according to claim 1, wherein glutathione and/or L-cysteine each are included to 0.01 to 5.0 wt% of the total weight of the oil-based skin treatment composition.

3. The oil-based skin treatment composition according to claim 1 or 2, further comprising an aluminum compound in powder form.
